(19) Europäisches Patentamt
European Patent Office
Office européen des brevets

(11) Publication number: 0 256 656 B1

(12) **EUROPEAN PATENT SPECIFICATION**

(45) Date of publication of patent specification: **23.09.92**

(51) Int. Cl.5: **A61K 7/50**, A61K 7/08, C11D 1/10, C11D 1/37, C11D 1/28, C11D 1/94

(21) Application number: **87306046.1**

(22) Date of filing: **08.07.87**

# (54) A liquid detergent composition.

(30) Priority: **11.07.86 US 884687**

(43) Date of publication of application: **24.02.88 Bulletin 88/08**

(45) Publication of the grant of the patent: **23.09.92 Bulletin 92/39**

(84) Designated Contracting States: **AT BE CH DE FR GB GR IT LI LU NL SE**

(56) References cited:
**EP-A- 0 068 352**
**EP-A- 0 075 906**
**EP-A- 0 081 691**
**GB-A- 751 273**
**GB-A- 1 466 560**
**US-A- 3 766 068**

(73) Proprietor: **THE PROCTER & GAMBLE COMPANY**
**One Procter & Gamble Plaza**
**Cincinnati Ohio 45202(US)**

(72) Inventor: **Maile, Robert Joseph. Jr.**
**6865 Esther Lane**
**Cincinnati, OH 45243(US)**

(74) Representative: **Brooks, Maxim Courtney et al**
**Procter & Gamble (NTC) Limited Whitley Road Longbenton**
**Newcastle-upon-Tyne NE12 9TS(GB)**

EP 0 256 656 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention concerns liquid detergent compositions which are mild, provide a lather of aesthetically pleasing character, and comprise a particular mixture of surfactants.

Liquid detergent compositions, such as hand cleaners, shower or bath cleaners, and shampoos, are staple items of commerce in modern societies. They are used by consumers to remove soil from the hands, the body, and the hair. If such products are to find wide acceptance among consumers, they must be mild (i.e. they must not be harsh or irritating to the skin), they must clean effectively, and they must possess pleasing aesthetic attributes or characteristics. Among the more important aesthetic attributes is the character of lather produced by the composition during use, with lather of high volume and exceptional creaminess being particularly desirable.

A number of references disclose detergent compositions. For example, Japanese OPI 61-63198, published December 15, 1981, discloses mixtures of anionic sulfate surfactants and betaine surfactants. US-A-3,980,769 issued September 14, 1976, to Ghilardi et al., discloses detergent compositions containing a mixture of anionic and amphoteric surfactants. US-A-4,110,263 issued August 29, 1978 to Lindemann et al., also discloses detergent compositions containing anionic and amphoteric surfactants. US-A-4,310,433 issued January 12, 1982, to Stiros discloses an aqueous liquid detergent composition comprising saturated and unsaturated potassium soaps and free fatty acid. US-A-4,472,297 issued September 18, 1984, to Bolich, Jr. et al., discloses liquid detergents which are cosmetically attractive and which contain a hydroxyl propyl guar gum, an alcohol, a surfactant, water and an electrolyte as essential components. US-A-3,085,067 issued April 9, 1963 to Anderson discloses a sarcosinate-based detergent composition formulated from a combination of ingredients so selected and so proportioned that outstanding lathering and other characteristics are obtained. US-A-3,766,068 discloses aqueous lubricating compositions consisting of an aqueous carrier containing a mixture of N-acylsarcosine and N-acyl taurine surfactants. GB-A-751273 discloses all-purpose detergent compositions comprising an amphoteric taurine surfactant together with N-acyl sarcosine and alkyl sulfate surfactants.

Accordingly, it is an object of the present invention to provide a liquid detergent composition which is mild and which possesses exemplary lathering characteristics in use.

It is a further object of this invention to provide a liquid detergent composition comprising a mixture of a taurate surfactant and a sarcosinate surfactant in combination with an auxiliary surfactant.

According to the present invention, there is provided a liquid detergent composition comprising:

(a) from 0.5% to 10% by weight of at least one taurate surfactant selected from salts of $C_{10}$-$C_{20}$ fatty acids of N-methyl taurine;

(b) from 0.5% to 10% by weight of at least one sarcosinate surfactant selected from salts of condensation products of $C_{10}$-$C_{20}$ fatty acids with sarcosine; and

(c) from 1% to 30% by weight of at least one auxiliary surfactant selected from salts of sulfate esters of polyethylene glycol ethers of fatty alcohols, alkyl sulfate surfactants, and mixtures thereof.

wherein the weight ratio of said taurate surfactant to said sarcosinate surfactant is from 1:3 to 3:1 and wherein the total level of surfactant present is from 2% to 40% by weight.

It has been surprisingly discovered that such detergent compositions comprising these mixtures of taurate and sarcosinate surfactants are mild and exhibit unexpected volumes of lather possessing unexpected creaminess and silky skin feel as compared to compositions comprising only one or the other of these surfactants.

As used herein, unless otherwise stated, all ratios are by weight and all percentages are by weight of the composition.

While this specification concludes with claims particularly pointing out and distinctly claiming the present invention, it is believed that the invention can be more readily understood from a reading of the following detailed description and the appended nonlimiting claims.

The liquid detergent composition comprises at least one taurate surfactant, at least one sarcosinate surfactant, and an auxiliary surfactant.

The essential components of the present invention are discussed in the following paragraphs.

### Taurate Surfactants

The taurate surfactants useful in the present invention are the salts of fatty acid amides of N-methyltaurine. They conform generally to the structural formula:

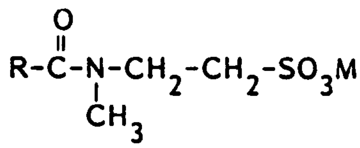

where

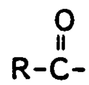

represents a fatty acid radical and M represents sodium, potassium, ammonium, or triethanolamine. Fatty acids having carbon chain lengths of from 10 to 20, including those derived from coconut, palm and tall oil, are used. Preferably, the fatty acid is derived from coconut. Preferably, sodium salts are used. The most preferred taurate is sodium methyl cocoyl taurate. This taurate surfactant is sold under the trademark Igepon TC-42 by GAF Corporation of New York, New York. The taurate surfactant is present at from 0.5% to 10% of the detergent composition.

<u>Sarcosinate Surfactants</u>

The sarcosinate surfactants useful in the present invention are N-acyl sarcosinates which are salts of condensation products of fatty acids with sarcosine. They conform generally to the structural formula:

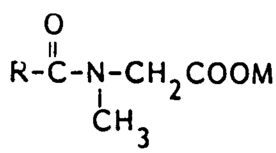

where, as above,

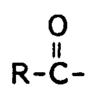

represents a fatty acid radical and M represents sodium, potassium, ammonium, or triethanolamine. Fatty acids having carbon chain lengths of from 10 to 20, including those derived from coconut, palm, and tall oil, are used. Preferably, the fatty acid is lauric acid. Preferably, sodium salts are used. The most preferred sarcosinate is sodium lauroyl sarcosinate. This sarcosinate surfactant is sold under the trademarks Hamposyl (RTM) L-30 and Hamposyl (RTM) L-95 by W. R. Grace & Co. of Lexington, Maine. The sarcosinate surfactant is present at from 0.5% to 10% of the detergent composition.

The taurate and sarcosinate surfactants are present in the composition at such levels that the weight ratio of taurate to sarcosinate is from 1:3 to 3:1.

<u>Auxiliary Surfactant</u>

Especially preferred as auxiliary surfactant are the salts of sulfate esters of the polyethylene glycol ethers of fatty alcohols; these materials have the general structural formula:

$R'(OCH_2CH_2)_nOSO_3M$

In this formula, R' represents the fatty alcohol moiety; M represents, as before, sodium, potassium, ammonium, or triethanolamine; and n is a number between 1 and about 15. Preferably, the fatty alcohol is

lauric alcohol. Preferably, M is sodium. Preferably, n is between 1 and 4 with an average value of n of 3 being particularly preferred. An especially preferred auxiliary surfactant is sodium laureth sulfate.

Also suitable are alkyl sulfate surfactants. These materials have the following general structural formula:

$R' OSO_3M$

As before, R' represents a fatty alcohol moiety and M represents sodium, potassium, ammonium, or triethanolamine. Fatty alcohols having carbon chain lengths of from about 10 to about 20, including those derived from coconut, palm oil and tall oil, can be used. Preferably, the fatty alcohol is lauryl alcohol. Preferably, sodium salts are used. The most preferred alkyl sulfate is sodium lauryl sulfate.

Mixtures of auxiliary surfactants can be used.

Preferably, the auxiliary surfactant is present at a level of from 1% to 30%.

Water

Water is a necessary component of the present invention. The water is preferably deionized and is preferably used at levels of from 60% to 98%.

Optional Components

The compositions herein can contain a variety of non-essential optional components suitable for rendering such compositions more acceptable to consumers. Such conventional optional ingredients are well known to those skilled in the art. They include preservatives such as benzyl alcohol, methyl paraben, propyl paraben, and imidazolidinyl urea; thickeners and viscosity modifiers such as the diethanolamide of long chain fatty acids (e.g., PEG-3 lauramide), block polymers of ethylene oxide and propylene oxide (such as Pluronic (RTM) F-88 offered by BASF Wyandotte of Wyandotte, Michigan), sodium chloride, sodium sulfate, polyvinyl alcohol, and ethyl alcohol; pH adjusting agents such as citric acid, succinic acid, phosphoric acid, sodium hydroxide, sodium carbonate, etc.; silicone fluids; perfumes; dyes; hydrotropes such as ammonium xylene sulfonate and potassium toluene sulfonate; sequestering agents such as disodium ethylenediamine tetraacetate; lather modifiers such as coconut monoethanolamide; any of the various deodorant systems used in personal care products, such as triclocarban (TCC) and triclosan (Irgasan (RTM) DP-300); and polymers such as polyquaternium-10 (UCARE (RTM) Polymer JR-400 as made by Union Carbide Corporation of Danbury, Connecticut), hydroxylpropyl guar (Jaguar (RTM) HP-60 as made by Celanese Plastics and Specialities Company of Louisville Kentucky), guar hydroxypropyltrimonium chloride (Jaguar (RTM) C-14S), polyquaternium-7 (Merquat (RTM) 550 as made by Merck & Company, Inc. of Rahway, New Jersey), and hydroxyethylcellulose. Such optional components are used individually at levels of from about 0.01% to about 10%, preferably from about 0.1% to about 5% by weight of the composition.

The pH of the present composition is not critical, but generally is in the range of from about 4 to about 8.

Methods and Manufacture

Any convenient method of manufacture as is commonly used in the industry, such as mixing the ingredients in a simple batch operation, can be used.

Form of Product

The compositions of this invention are usually supplied as pourable liquids in containers ready for use by consumers. In this situation, the consumer dispenses the liquid as a liquid from the container and uses it with water to clean his or her hands, body, or hair.

Optionally, the compositions can be supplied as aerosol products as well known by those skilled in the art. In this particular situation, the product is dispensed as a foam and is then used by the consumer with water to clean his or her hands, body, or hair. In aerosol products, the taurate and sarcosinate surfactants are each preferably present at from 0.1% to 7.5% and the auxiliary surfactant at from 1% to 20%; the total surfactant present is preferably present at from 2% to 30% of the composition. The above noted ratio of taurate surfactant to sarcosinate surfactant must be maintained. Water preferably comprises from 70% to 98% of the composition. These aerosol products also incorporate any of the various propellants (such as

hydrocarbons, fluorocarbons, nitrous oxide, etc.) well known to those skilled in the art.

Optionally, the compositions can be supplied as pastes as well known to those skilled in the art. In this particular situation, the product is dispensed, as from a tube, and used by the consumers in a manner similar to a liquid product to clean his or her hands, body, or hair.

The following example is presented by way of illustration and not by way of limitation.

EXAMPLE

| | |
|---|---|
| Sodium laureth sulfate | 10.00% |
| Sodium methyl cocoyl taurate | 1.8 |
| Sodium lauroyl sarcosinate | 2.25 |
| Coconut monoethanolamide | 4.00 |
| Opacifier | 1.00 |
| Citric Acid | 0.25 |
| EDTA | 0.1 |
| Irgasan (RTM) DP-300 | 0.3 |
| TCC | 0.15 |
| Dye | 0.01 |
| UCARE (RTM) JR-400 | 0.75 |
| NaCl | 0.3 |
| Perfume | 0.12 |
| Glycerine | 1.0 |
| Water | qs100 |

The various components are mixed to form a liquid detergent composition. When this composition is used by a human consumer as a hand cleansing product, it cleans the user's hands in an efficient manner and, during use, produces a high volume of extraordinarily creamy lather.

## Claims

**1.** A liquid detergent composition comprising:

(a) from 0.5% to 10% by weight of at least one taurate surfactant selected from salts of $C_{10}$-$C_{20}$ fatty acids of N-methyl taurine;

(b) from 0.5% to 10% by weight of at least one sarcosinate surfactant selected from salts of condensation products of $C_{10}$-$C_{20}$ fatty acids with sarcosine; and

(c) from 1% to 30% by weight of at least one auxiliary surfactant selected from salts of sulfate esters of polyethylene glycol ethers of fatty alcohols, alkyl sulfate surfactants, and mixtures thereof;

wherein the weight ratio of said taurate surfactant to said sarcosinate surfactant is from 1:3 to 3:1 and wherein the total level of surfactant present is from 2% to 40% by weight.

**2.** A detergent composition according to Claim 1 wherein said taurate is sodium methyl cocoyl taurate.

**3.** A detergent composition according to Claim 1 or 2 wherein said sarcosinate is sodium lauroyl sarcosinate.

**4.** A liquid detergent composition according to Claim 1 comprising:

(a) from 0.5% to 10% by weight of sodium methyl cocoyl taurate;

(b) from 0.5% to 10% by weight of sodium lauroyl sarcosinate; and

(c) from 1% to 30% by weight of sodium laureth sulfate

**5.** An aerosol detergent composition according to Claim 1 comprising:

(a) from 0.1% to 7.5% by weight of taurate surfactant;

(b) from 0.1% to 7.5% by weight of sarcosinate surfactant;

(c) from 1% to 20% by weight of auxiliary surfactant;

wherein the total level of surfactant present is from 2% to 30% by weight.

**Patentansprüche**

1. Flüssige Detergenszusammensetzung, umfassend:
   (a) von 0,5 Gew.-% bis 10 Gew.-% an mindestens einem Taurat-grenzflächenaktiven Mittel, ausgewählt unter Salzen von $C_{10}$-$C_{20}$-Fettsäuren von N-Methyltaurin;
   (b) von 0,5 Gew.-% bis 10 Gew.-% an mindestens einem Sarcosinat-grenzflächenaktiven Mittel, ausgewählt unter Salzen von Kondensationsprodukten von $C_{10}$-$C_{20}$-Fettsäuren mit Sarcosin; und
   (c) von 1 Gew.-% bis 30 Gew.-% an mindestens einem grenzflächenaktiven Hilfsmittel, ausgewählt unter Salzen von Sulfatestern von Polyethylenglykolethern von Fettalkoholen, Alkylsulfat-grenzflächenaktiven Mitteln und Gemischen hievon;
   worin das Gewichtsverhältnis von dem genannten Taurat-grenzflächenaktiven Mittel zu dem genannten Sarcosinat-grenzflächenaktiven Mittel von 1:3 bis 3:1 beträgt und worin die Gesamtmenge an vorhandenem grenzflächenaktivem Mittel von 2 Gew.-% bis 40 Gew.-% beträgt.

2. Detergenszusammensetzung nach Anspruch 1, worin das genannte Taurat Natriummethylcocoyltaurat ist.

3. Detergenszusammensetzung nach Anspruch 1 oder 2, worin das genannte Sarcosinat Natriumlauroylsarcosinat ist.

4. Flüssige Detergenszusammensetzung nach Anspruch 1, umfassend:
   (a) von 0,5 Gew.-% bis 10 Gew.-% an Natriummethylcocoyltaurat;
   (b) von 0,5 Gew.-% bis 10 Gew.-% an Natriumlauroylsarcosinat; und
   (c) von 1 Gew.-% bis 30 Gew.-% an Natriumlaurethsulfat.

5. Aerosoldetergenszusammensetzung nach Anspruch 1, umfassend:
   (a) von 0,1 Gew.-% bis 7,5 Gew.-% an Taurat-grenzflächenaktivem Mittel;
   (b) von 0,1 Gew.-% bis 7,5 Gew.-% an Sarcosinat-grenzflächenaktivem Mittel;
   (c) von 1 Gew.-% bis 20 Gew.-% an grenzflächenaktivem Hilfsmittel;
   worin die Gesamtmenge an vorhandenem grenzflächenaktivem Mittel von 2 Gew.-% bis 30 Gew.-% beträgt.

**Revendications**

1. Composition détergente liquide, comprenant :
   (a) de 0,5% à 10% en poids d'au moins un tensioactif taurate choisi parmi les sels d'acides gras en $C_{10}$-$C_{20}$ de N-méthyltaurine;
   (b) de 0,5% à 10% en poids d'au moins un tensioactif sarcosinate choisi parmi les sels de produits de condensation d'acides gras en $C_{10}$-$C_{20}$ et de sarcosine; et
   (c) de 1% à 30% en poids d'au moins un tensioactif auxiliaire choisi parmi les sels esters sulfates d'éthers de polyéthylèneglycol et d'alcools gras, les tensioactifs alkylsulfates, et leurs mélanges;
   dans laquelle le rapport pondéral dudit tensioactif taurate audit tensioactif sarcosinate est de 1:3 à 3:1 et dans laquelle la proportion totale de tensioactif présent est de 2% à 40% en poids.

2. Composition détergente selon la revendication 1, dans laquelle ledit taurate est le méthyl-alkyl de coprah-taurate de sodium.

3. Composition détergente selon la revendication 1 ou 2, dans laquelle ledit sarcosinate est le lauroylsarcosinate de sodium.

4. Composition détergente liquide selon la revendication 1, comprenant :
   (a) de 0,5% à 10% en poids de méthyl-alkyl de coprah-taurate de sodium;
   (b) de 0,5% à 10% en poids de lauroylsarcosinate de sodium; et
   (c) de 1% à 30% en poids de laureth-sulfate de sodium.

5. Composition détergente en aérosol selon la revendication 1, comprenant :
   (a) de 0,1% à 7,5% en poids de tensioactif taurate;
   (b) de 0,1% à 7,5% en poids de tensioactif sarcosinate;

(c) de 1% à 20% en poids de tensioactif auxiliaire;

dans laquelle la proportion totale de tensioactif présent est de 2% à 30% en poids.